(19) Europäisches Patentamt
European Patent Office
Office européen des brevets

(11) **EP 2 383 564 A1**

(12) **EUROPEAN PATENT APPLICATION**
published in accordance with Art. 153(4) EPC

(43) Date of publication:
**02.11.2011 Bulletin 2011/44**

(51) Int Cl.:
***G01N 21/64*** *(2006.01)*

(21) Application number: **10729151.0**

(22) Date of filing: **04.01.2010**

(86) International application number:
**PCT/JP2010/000002**

(87) International publication number:
**WO 2010/079731 (15.07.2010 Gazette 2010/28)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **09.01.2009 JP 2009003414**

(71) Applicant: **Mitsui Engineering & Shipbuilding Co., Ltd.**
**Chuo-ku**
**Tokyo 104-8439 (JP)**

(72) Inventor: **DOI, Kyouji**
**Tamano-shi**
**Okayama 706-8651 (JP)**

(74) Representative: **Hards, Andrew**
**Hards & Franke**
**Patentanwälte Partnerschaft**
**Widenmayerstraße 25**
**80538 München (DE)**

(54) **FLUORESCENCE DETECTING DEVICE AND FLUORESCENCE DETECTING METHOD**

(57) When a fluorescence relaxation time of a fluorochrome is determined using a measurement object obtained by attaching the fluorochrome to an analyte, a first laser beam is intensity-modulated by a modulation signal with a frequency of $f_1$ and a second laser beam is intensity-modulated by a modulation signal with a frequency of $f_2$, and these laser beams are emitted. A first fluorescent signal and a second fluorescent signal are obtained by receiving fluorescence within a first wavelength band and a second wavelength band emitted by the measurement object irradiated with the laser beams. The first fluorescent signal is mixed with the modulation signal with a frequency of $f_1$ to produce first fluorescence data $P_1$, and the second fluorescent signal is mixed with the modulation signal with a frequency of $f_2$ to produce second fluorescence data $P_2$. The fluorescence relaxation time of the fluorochrome is calculated using fluorescence data obtained by subtracting the result of multiplication of the second fluorescence data $P_2$ by a second constant from the result of multiplication of the first fluorescence data $P_1$ by a first constant.

FIG.5

EP 2 383 564 A1

## Description

TECHNICAL FIELD

The present invention relates to a device and a method for detecting fluorescence by processing a fluorescent signal of fluorescence emitted by a measurement object, which is an analyte having a fluorochrome attached thereto, by irradiation with laser light.

BACKGROUND ART

In the medical and biological fields, flow cytometers are widely used. A flow cytometer analyzes the type, frequency, and characteristics of a measurement object such as cells or genes by allowing a photoelectric converter such as a photomultiplier or an avalanche photodiode to receive fluorescence emitted by the measurement object irradiated with laser light.

More specifically, in a flow cytometer, a measurement object obtained by labeling an analyte such as a biological material (e.g., cells, DNA, RNA, enzymes, or proteins) with a fluorescent reagent is allowed to flow through a tube together with a sheath liquid flowing under pressure at a speed of about 10 m/s or less so that a laminar sheath flow is formed. When the measurement object in the laminar sheath flow is irradiated with laser light by the flow cytometer, the flow cytometer receives fluorescence emitted by a fluorochrome attached to the analyte and identifies the analyte by using the fluorescence as a label.

The flow cytometer can measure the relative amounts of, for example, DNA, RNA, enzymes, proteins etc. contained in a cell, and also can quickly analyze their functions. Further, a cell sorter or the like is used to selectively collect only a predetermined type of cells or chromosomes, which have been identified by the flow cytometer based on fluorescence, alive quickly.

The use of the flow cytometer is required to quickly identify more kinds of measurement objects with high accuracy based on information about fluorescence.

Japanese Patent Application Laid-Open No. 2006-226698 discloses a fluorescence detecting device and a fluorescence detecting method which are capable of quickly and accurately identifying many kinds of measurement objects by calculating the fluorescence lifetime (fluorescence relaxation time) of fluorescence emitted by a fluorochrome bound to a measurement object irradiated with laser light.

Japanese Patent Application Laid-Open No. 2006-226698 describes that the fluorescence detecting device determines the phase delay of a fluorescent signal of fluorescence emitted by a measurement object irradiated with intensity-modulated laser light with respect to a modulation signal used to modulate the intensity of the laser light, and further calculates a fluorescence relaxation time from the phase delay.

However, fluorescence emitted by a measurement object includes not only fluorescence emitted by a fluorochrome bound to an analyte such as a cell but also autofluorescence emitted by the analyte itself such as a cell. In most cases, the fluorescence intensity of autofluorescence is lower than that of fluorescence emitted by a fluorochrome, but there is a case where the fluorescence intensity of received fluorescence which is emitted by a fluorochrome is also low. In such a case, there is a problem that the influence of received autofluorescence cannot be ignored and therefore it is impossible to accurately calculate a phase delay and a fluorescence relaxation time.

DISCLOSURE OF THE INVENTION

Problems to be Solved by the Invention

In order to solve the above problem, it is an object of the present invention to provide a fluorescence detecting device and a fluorescence detecting method which make it possible to calculate a fluorescence relaxation time with high accuracy by processing a fluorescent signal of fluorescence emitted by a measurement object, which is an analyte having a fluorochrome attached thereto, by irradiation with laser light.

Means for Solving the Problems

One aspect of the present invention provides a device for detecting fluorescence by processing a fluorescent signal of fluorescence emitted by a measurement object, which is an analyte having at least one fluorochrome attached thereto, by irradiation with laser light, the device including:

a light source unit that modulates an intensity of a first laser beam by a modulation signal having a frequency of $f_1$ and modulates an intensity of a second laser beam by a modulation signal having a frequency of $f_2$ different from

$f_1$ to emit the modulated first laser beam and the modulated second laser beam;

a light-receiving unit that includes a first light-receiving element that receives, within a first wavelength band, fluorescence emitted by the measurement object irradiated with the first laser beam and the second laser beam to output a first fluorescent signal and a second light-receiving element that receives, within a second wavelength band different from the first wavelength band, fluorescence emitted by the measurement object irradiated with the first laser beam and the second laser beam to output a second fluorescent signal;

a first processing unit that produces, by mixing the first fluorescent signal with the modulation signal having a frequency of $f_1$, first fluorescence data $P_1$ containing information about phase and intensity and produces, by mixing the second fluorescent signal with the modulation signal having a frequency of $f_2$, second fluorescence data $P_2$ containing information about phase and intensity; and

a second processing unit that calculates a fluorescence relaxation time of the fluorochrome using fluorescence data obtained by subtracting a result obtained by multiplying the second fluorescence data $P_2$ by a second constant from a result obtained by multiplying the first fluorescence data $P_1$ by a first constant.

Another aspect of the present invention provides a method for detecting fluorescence by processing a fluorescent signal of fluorescence emitted by a measurement object, which is an analyte having a fluorochrome attached thereto, by irradiation with laser light, the method including the steps of:

modulating an intensity of a first laser beam by a modulation signal having a frequency of $f_1$ and modulating an intensity of a second laser beam by a modulation signal having a frequency of $f_2$ different from $f_1$ to emit the modulated first laser beam and the modulated second laser beam;

receiving, within a first wavelength band, fluorescence emitted by the measurement object irradiated with the first laser beam and the second laser beam to output a first fluorescent signal and receiving, within a second wavelength band different from the first wavelength band, fluorescence emitted by the measurement object irradiated with the first laser beam and the second laser beam to output a second fluorescent signal;

producing, by mixing the first fluorescent signal with the modulation signal having a frequency of $f_1$, first fluorescence data $P_1$ containing information about phase and intensity and producing, by mixing the second fluorescent signal with the modulation signal having a frequency of $f_2$, second fluorescence data $P_2$ containing information about phase and intensity; and

calculating a fluorescence relaxation time of the fluorochrome using fluorescence data obtained by subtracting a result obtained by multiplying the second fluorescence data $P_2$ by a second constant from a result obtained by multiplying the first fluorescence data $P_1$ by a first constant.

EFFECTS OF THE INVENTION

The fluorescence detecting device and the fluorescence detecting method according to the above aspects of the present invention make it possible to calculate a fluorescence relaxation time with high accuracy by processing a fluorescent signal of fluorescence emitted by a measurement object irradiated with laser light.

BRIEF DESCRIPTION OF THE DRAWINGS

Fig. 1 is a schematic diagram illustrating one example of the structure of a flow cytometer that uses a fluorescence detecting device according to the present invention using intensity-modulated laser light.
Fig. 2 is a schematic diagram illustrating the structure of one example of a light source unit used in the flow cytometer illustrated in Fig. 1.
Fig. 3 is a schematic diagram illustrating the structure of one example of a light-receiving unit used in the flow cytometer illustrated in Fig. 1.
Fig. 4 is a schematic diagram illustrating the structure of one example of a control/processing unit used in the flow cytometer illustrated in Fig. 1.
Fig. 5 is a schematic diagram illustrating the structure of one example of an analyzing device used in the flow cytometer illustrated in Fig. 1.

DESCRIPTION OF THE REFERENCE NUMERALS

| | |
|---|---|
| 10 | flow cytometer |
| 12 | sample |
| 20 | signal processing device |
| 22 | laser light source unit |
| 22a, 22b | light source |
| 23a, 26b | dichroic mirror |
| 23b, 26a | lens system |
| 24, 26 | light-receiving unit |
| $26c_1$, $26c_2$ | band-pass filter |
| 27a, 27b | photoelectric converter |
| 28 | control/processing unit |
| 30 | tube |
| 32 | collection vessel |
| 34a, 34b | laser driver |
| 40 | signal generation unit |
| 42 | signal processing unit |
| 44 | signal control unit |
| 46a, 46b | oscillator |
| 48a, 48b | power splitter |
| 50a, 50b, 52a, 52b, 54a, 54b, 64 | amplifier |
| 58a, 58b | IQ mixer |
| 60 | system controller |
| 62 | low-pass filter |
| 66 | A/D converter |
| 80 | analyzing device |
| 82 | memory |
| 84 | frequency analyzing unit |
| 86 | autofluorescence removing unit |
| 88 | fluorescence data correcting unit |
| 90 | fluorescence intensity calculating unit |
| 92 | phase delay calculating unit |
| 94 | fluorescence relaxation time calculating unit |

DESCRIPTION OF EMBODIMENTS

Hereinbelow, the present invention will be described in detail based on a flow cytometer appropriately employing a fluorescence detecting device according to the present invention for detecting fluorescence emitted by irradiation with intensity-modulated laser light.

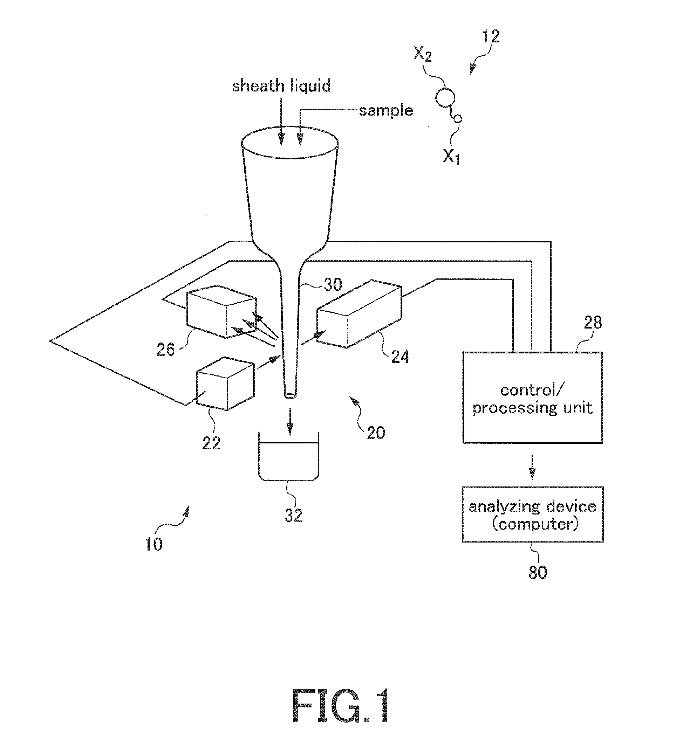

Fig. 1 is a schematic diagram illustrating the structure of a flow cytometer 10 that employs a fluorescence detecting device according to one embodiment of the present invention using intensity-modulated laser light.

The flow cytometer 10 includes a signal processing device 20 and an analyzing device (computer) 80. The signal processing device 20 detects and processes a fluorescent signal of fluorescence emitted by a sample 12, which is a measurement object, by irradiation with laser light. The analyzing device (computer) 80 calculates a fluorescence intensity and a fluorescence relaxation time from processing results obtained by the signal processing device 20. The following description is made with reference to a case where a measurement object composed of a cell (analyte) $X_2$ and a fluorescent protein (fluorochrome) $X_1$ attached to the cell $X_2$ is used as an example of the sample 12. In this embodiment, a fluorochrome may be used instead of the fluorescent protein. The analyte is not limited to a cell, and may be, for example, a biological material, such as DNA, RNA, enzymes, or proteins, or an artificially-produced microbead that emits fluorescence. It is to be noted that in Fig. 1, the structure of the sample 12 is schematically illustrated, in which the fluorescent protein $X_1$ is attached to the cell $X_2$ by a string or the like. However, the sample 12 may have, for example, a structure in which the fluorescent protein $X_1$ is introduced into and dispersed in the cell $X_2$. The number of the fluorescent proteins $X_1$ attached to the cell $X_2$ is not limited to one, and may be two or more.

The signal processing device 20 includes a laser light source unit 22, light-receiving units 24 and 26, a control/ processing unit 28, and a tube 30.

The control/processing unit 28 includes a control unit that modulates the intensity of laser light emitted from the laser light source unit 22 at a predetermined frequency and a signal processing unit that processes a fluorescent signal from the sample 12. The tube 30 allows the samples 12 to flow therethrough together with a sheath liquid forming a high-speed flow so that a laminar sheath flow is formed.

A collection vessel 32 is provided at the outlet of the tube 30. The flow cytometer 10 may include a cell sorter for separating a biological material, such as predetermined cells, in the samples 12 over a short time after irradiation with laser light to collect the biological material in different collection vessels.

The laser light source unit 22 is a unit that emits two laser beams different in wavelength. A lens system is provided so that the laser beams are focused on a predetermined position in the tube 30. The focus position is defined as a measurement point where the sample 12 is measured.

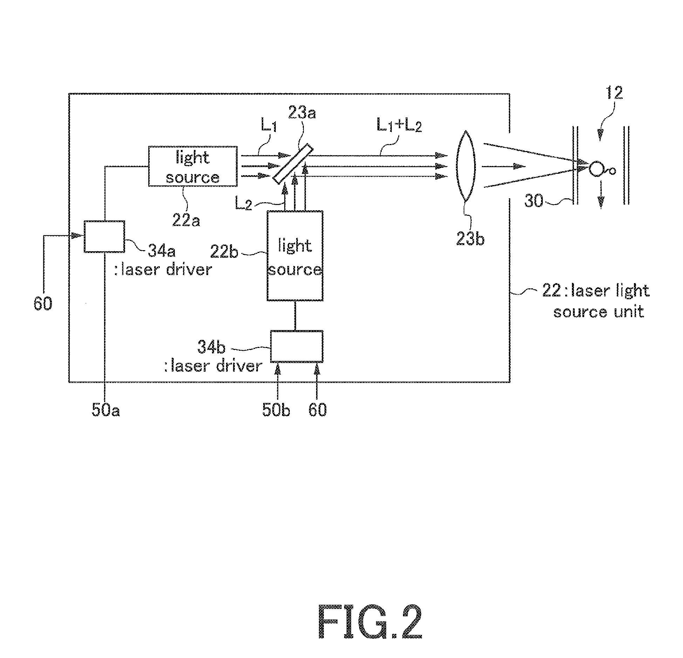

Fig. 2 is a diagram illustrating one example of the structure of the laser light source unit 22.

The laser light source unit 22 emits intensity-modulated laser beams having wavelengths within a visible light band. The laser light source unit 22 includes a light source 22a and a light source 22b. The light source 22a emits a first laser beam $L_1$ having a wavelength within a wavelength band, in which the optical absorption (optical-absorption coefficient) of the fluorescent protein $X_1$ is higher than that of the cell (analyte) $X_2$, as a CW (continuous-wave) laser beam while modulating the intensity of the CW laser beam $L_1$ by a modulating signal having a predetermined frequency of $f_1$. The light source 22b emits a second laser beam $L_2$ having a wavelength within a wavelength band, in which the optical absorption of the fluorescent protein $X_1$ is lower than that of the cell (analyte) $X_2$, as a CW (continuous-wave) laser beam while modulating the intensity of the CW laser beam $L_2$ by a modulation signal having a predetermined frequency of $f_2$.

The laser light source unit 22 further includes a dichroic mirror 23a, a lens system 23b, and laser drivers 34a and 34b. It is to be noted that a half mirror may be used instead of the dichroic mirror 23a.

The dichroic mirror 23a transmits laser light of wavelengths within a specific wavelength band and reflects laser light of wavelengths outside the specific wavelength band. The lens system 23b focuses laser light $L_1 + L_2$ composed of the laser beam $L_1$ and the laser beam $L_2$ on the measurement point in the tube 30. The laser driver 34a drives the light source 22a and the laser driver 34b drives the light source 22b.

As the light sources that emit laser beams, for example, semiconductor lasers are employed. The laser beams have an output of, for example, about 5 to 100 mW

On the other hand, a frequency (modulation frequency) used to modulate the intensity of each of the laser beams $L_1$ and $L_2$ is, for example, 10 to 50 MHz, whose a cycle time of the modulation is slightly longer than a fluorescence relaxation time. A frequency used to modulate the intensity of the laser beam $L_1$ and a frequency used to modulate the intensity of the laser beam $L_2$ are different from each other. This allows the sample 12 excited by the laser beams to emit fluorescence of different frequencies so that the signal processing device 20 can determine which of the laser beams has induced the emission of received fluorescence, to separate information about fluorescence.

The dichroic mirror 23a is a mirror that transmits the laser beam $L_1$ and reflects the laser beam $L_2$. The laser beam $L_1$ and the laser beam $L_2$ are combined into one irradiation light by the dichroic mirror 23a, and the sample 12 is irradiated with the irradiation light at the measurement point.

The light sources 22a and 22b oscillate at predetermined wavelength bands so that a fluorochrome is excited by the laser beams $L_1$ and $L_2$ and emit fluorescence of specific wavelength bands. When passing through the measurement point in the tube 30, the sample 12 is irradiated with and excited by the laser beams $L_1$ and $L_2$ so that the fluorescent protein $X_1$ emits fluorescence at specific wavelengths. At this time, the cell $X_2$ emits autofluorescence.

The light-receiving unit 24 is arranged so as to be opposed to the laser light source unit 22 with the tube 30 being provided therebetween. The light-receiving unit 24 is equipped with a photoelectric converter that receives laser light forward-scattered by the sample 12 passing through the measurement point and outputs a detection signal indicating the passage of the sample 12 through the measurement point. The detection signal outputted from the light-receiving unit 24 is supplied to the control/processing unit 28 and the analyzing device 80 and is used as a trigger signal to announce the timing of passage of the sample 12 through the measurement point in the tube 30 and as an ON signal for controlling the start of processing or an OFF signal.

On the other hand, the light-receiving unit 26 is arranged in a direction perpendicular to a direction in which laser light emitted from the laser light source unit 22 travels and to a direction in which the samples 12 move in the tube 30. The light-receiving unit 26 is equipped with two or more photoelectric converters that receive fluorescence emitted by the sample 12 irradiated with laser light at the measurement point.

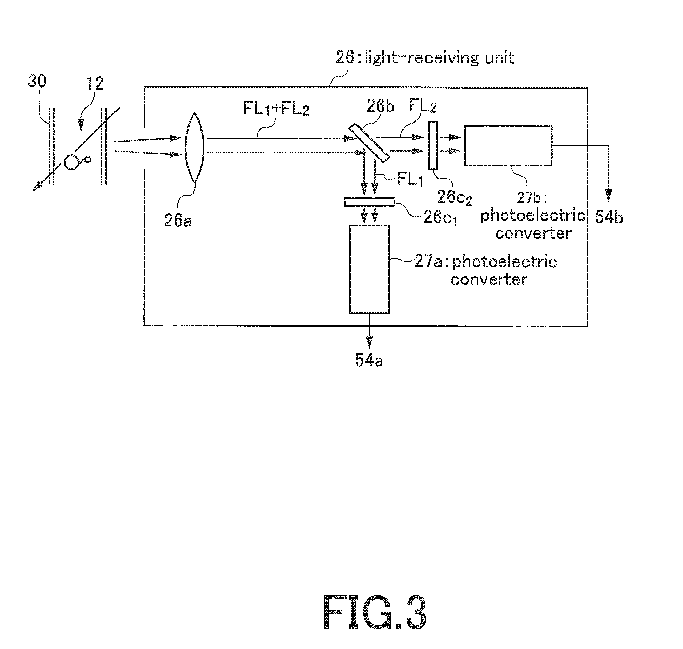

Fig. 3 is a schematic diagram illustrating the structure of one example of the light-receiving unit 26.

The light-receiving unit 26 illustrated in Fig. 3 includes a lens system 26a that focuses fluorescent signals from the sample 12, a dichroic mirror 26b, band-pass filters $26c_1$ and $26c_2$, and photoelectric converters (light-receiving elements) 27a and 27b such as photomultipliers.

The lens system 26a is configured to focus fluorescence received by the light-receiving unit 26 on the light-receiving surfaces of the photoelectric converters 27a and 27b.

The dichroic mirror 26b is a mirror that reflects fluorescence of wavelengths within a predetermined wavelength band but transmits fluorescence of wavelengths outside the predetermined wavelength band. The reflection wavelength band of the dichroic mirror 26b and the transmission wavelengths bands of the band-pass filters $26c_1$ and $26c_2$ are set so that fluorescence of a predetermined wavelength band can be received by the photoelectric converter 27a after filtering by the band-pass filter $26c_1$ and fluorescence of a predetermined wavelength band can be received by the photoelectric converter 27b after filtering by the band-pass filter $26c_2$.

The band-pass filter $26c_1$ is provided in front of the light-receiving surface of the photoelectric converter 27a and transmits only fluorescence of a predetermined wavelength band, and the band-pass filter $26c_2$ is provided in front of the light-receiving surface of the photoelectric converter 27b and transmits only fluorescence of a predetermined wavelength band. A wavelength band $R_1$ of fluorescence that can pass through one of the band-pass filter $26c_1$ is set so as to correspond to the wavelength band of fluorescence emitted by the fluorescent protein $X_1$, and a wavelength band $R_2$ of fluorescence that can pass through the other band-pass filter and $26c_2$ is set so as to correspond to the wavelength band of autofluorescence emitted by the cell $X_2$. The transmission wavelength band $R_1$ is, for example, a wavelength band ranging from 474 nm to 514 nm to mainly receive fluorescence emitted by irradiation with the laser beam $L_1$ of 408 nm emitted from the light source 22a. The transmission wavelength band $R_2$ is, for example a wavelength band ranging from 530 nm to 570 nm to mainly receive fluorescence emitted by irradiation with the laser beam $L_2$ of 455 nm emitted from the light source 22b.

In this case, the ratio of fluorescence intensity between fluorescence within the wavelength band $R_1$ emitted by the fluorescent protein $X_1$ and fluorescence within the wavelength band $R_1$ emitted by the cell $X_2$ is preferably different from the ratio of fluorescence intensity between fluorescence within the wavelength band $R_2$ emitted by the fluorescent protein $X_1$ and fluorescence within the wavelength band $R_2$ emitted by the cell $X_2$.

For example, the wavelength band $R_1$ is preferably set so as to correspond to the fluorescent protein $X_1$ so that when the sample 12 is irradiated with the laser beam $L_1$ the fluorescence intensity of fluorescence emitted by the fluorescent protein $X_1$ is higher than that of autofluorescence emitted by the cell $X_2$. At this time, the wavelength band $R_2$ is preferably set so as to correspond to autofluorescence so that when the sample 12 is irradiated with the laser beam $L_2$, the fluorescence intensity of autofluorescence emitted by the cell $X_2$ is higher than that of fluorescence emitted by the fluorescent protein $X_1$. The term "autofluorescence" used herein refers to fluorescence emitted by an object other than a fluorochrome of interest and causing background noise interfering with fluorescence measurement, and more specifically refers to fluorescence emitted by, for example, an undyed cell itself and having a spectrum extending over a broad wavelength band.

The photoelectric converters 27a and 27b are each a light-receiving element equipped with, for example, a photomultiplier as a sensor to convert light received by its photoelectric surface into an electric signal. Here, the emission of fluorescence to be received by each of the photoelectric converters is induced by excitation with laser light whose intensity is modulated at a predetermined frequency, and therefore a fluorescent signal outputted from each of the photoelectric converters is a signal whose intensity varies at the predetermined frequency. The fluorescent signal is supplied to the control/processing unit 28.

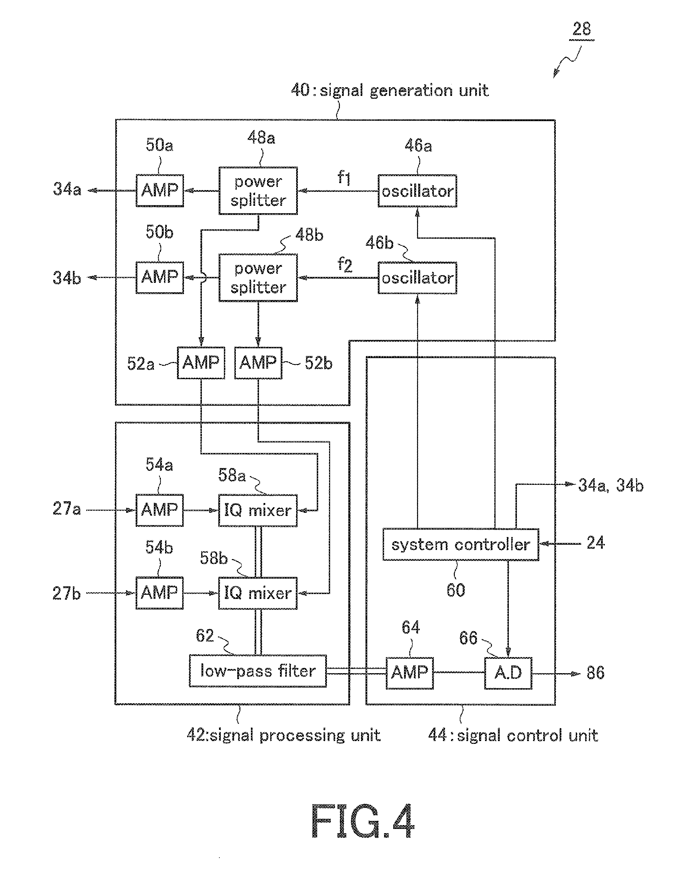

As illustrated in Fig. 4, the control/processing unit 28 includes a signal generation unit 40, a signal processing unit 42, and a signal control unit 44.

The signal generation unit 40 generates a modulation signal for modulating the intensity of the laser beam $L_1$ at a predetermined frequency and a modulation signal for modulating the intensity of the laser beam $L_2$ at a predetermined frequency.

More specifically, the signal generation unit 40 includes oscillators 46a and 46b, power splitters 48a and 48b, and amplifiers 50a, 50b, 52a, and 52b. The signal generation unit 40 supplies the modulation signal generated by the oscillator 46a and the modulation signal generated by the oscillator 46b to the laser drivers 34a and 34b of the laser light source unit 22, respectively, and supplies these modulation signals also to the signal processing unit 42. As will be described later, the modulation signals supplied to the signal processing unit 42 are used as reference signals for detecting fluorescent signals outputted from the photoelectric converters 27a and 27b. It is to be noted that each of the modulation signals is a signal obtained by superimposing a sinusoidal signal having a predetermined frequency on a DC component, and the frequency is set to a value in the range of 10 to 50 MHz. The oscillator 46a generates a signal having a frequency of $f_1$ and the oscillator 46b generates a signal having a frequency of $f_2$ different from $f_1$ so that modulation signals different in frequency are generated.

The signal processing unit 42 extracts, by using fluorescent signals outputted from the photoelectric converters 27a and 27b, information about phase delay of fluorescence emitted by irradiation with laser light. The signal processing unit 42 includes amplifiers 54a and 54b, IQ mixers 58a and 58b, and a low-pass filter 62. The amplifier 54a amplifies a fluorescent signal outputted from the photoelectric converter 27a, and the amplifier 54b amplifies a fluorescent signal outputted from the photoelectric converter 27b.

The IQ mixer 58a is a device that mixes the fluorescent signal supplied from the photoelectric converter 27a with the modulation signal supplied from the signal generation unit 40 as a reference signal, and the IQ mixer 58b is a device that mixes the fluorescent signal supplied from the photoelectric converter 27b with the modulation signal supplied from the signal generation unit 40 as a reference signal. More specifically, each of the IQ mixers 58a and 58b multiplies the reference signal by the fluorescent signal (RF signal) to generate an I signal containing a component of the fluorescent signal in phase with the modulation signal and a Q signal containing a component of the fluorescent signal shifted in phase by 90 degrees with respect to the modulation signal. The I signal containing an in-phase component is generated by mixing the modulation signal with the fluorescent signal, and the Q signal containing a component shifted in phase by 90 degrees is generated by mixing a signal obtained by shifting the phase of the modulation signal by 90 degrees with the fluorescent signal. This makes it possible to remove a fluorescent signal of fluorescence within the wavelength band $R_1$ emitted by excitation with the laser beam $L_2$ and a fluorescent signal of fluorescence within the wavelength band $R_2$ emitted by excitation with the laser beam $L_1$

The low-pass filter 62 is a unit that filters the I signal and the Q signal generated by each of the IQ mixers 58a and 58b to extract low-frequency signals. By performing the filtering, a component (Re component) of the fluorescent signal in phase with the modulation signal and a component (Im component) of the fluorescent signal shifted in phase by 90° with respect to the modulation signal are extracted as fluorescence data. The extracted components are sent to the signal control unit 44. The Re component and the Im component are obtained from both the wavelength bands $R_1$ and $R_2$ corresponding to the photoelectric converters 27a and 27b, and therefore a pair of the Re component and the Im component obtained from the wavelength band $R_1$ and a pair of the Re component and the Im component obtained from the wavelength band $R_2$ are sent to the signal control unit 44. Hereinafter, mixing performed by the IQ mixers 58a and 58b and filtering performed by the low-pass filter 62 are collectively referred to as "frequency-down conversion", and data obtained by frequency-down conversion is referred to as "fluorescence data".

The signal control unit 44 amplifies the Re component and the Im component of the fluorescent signal supplied from the signal processing unit 42, and performs A/D conversion.

More specifically, the signal control unit 44 includes a system controller 60 that gives instructions for controlling the operations of the individual units and manages all the operations of the flow cytometer 10, an amplifier 64 that amplifies the Re component and the Im component generated by the signal processing unit 42, and an A/D converter 66 that samples the amplified Re component and the amplified Im component.

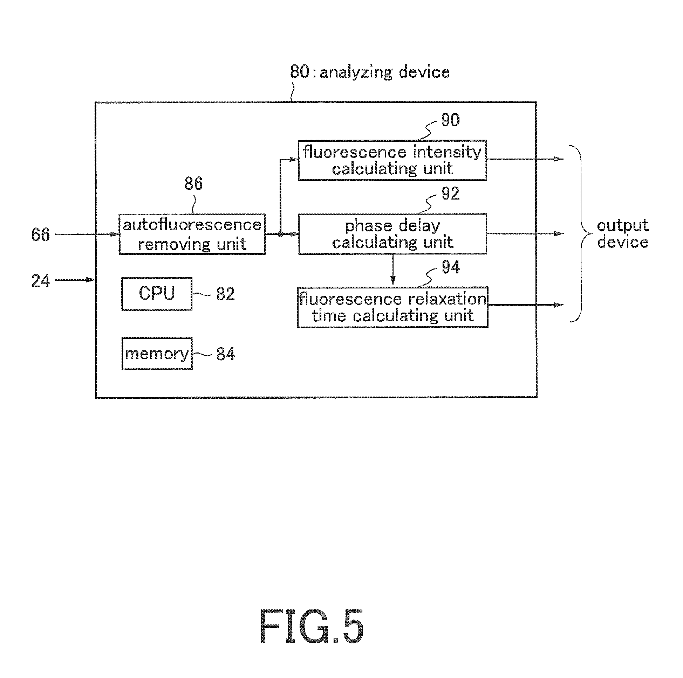

The analyzing device 80 determines, from the A/D converted Re component and the A/D converted Im component obtained by the signal control unit 44, the phase delay angle of fluorescence with respect to the laser beam, and further determines, from the phase delay angle, a fluorescence relaxation time constant (fluorescence relaxation time) and a fluorescence intensity. Fig. 5 is a schematic diagram illustrating the structure of the analyzing device 80. The analyzing device 80 is constituted of a computer including a CPU 82 and a memory 84. The analyzing device 80 further includes an autofluorescence removing unit 86, a fluorescence intensity calculating unit 90, a phase delay calculating unit 92, and a fluorescence relaxation time calculating unit 94. These units are software modules performing their functions by executing software stored in the computer, but can be, of course, provided by dedicated circuits.

The autofluorescence removing unit 86 is a unit that calculates, by using the Re components and the Im components supplied from the signal control unit 44, fluorescence data of fluorescence emitted by the fluorescent protein $X_1$ by removing autofluorescence emitted by the cell $X_2$. The Re components and the Im components supplied to the analyzing device 80 include information obtained by receiving fluorescence within the wavelength band $R_1$ emitted by the fluorescent protein $X_1$ by excitation with the laser beam $L_1$ and autofluorescence within the wavelength band $R_1$ emitted by the cell $X_2$ by excitation with the laser beam $L_1$ and information obtained by receiving fluorescence within the wavelength band $R_2$ emitted by the fluorescent protein $X_1$ by excitation with the laser beam $L_2$ and autofluorescence within the wavelength band $R_2$ emitted by the cell $X_2$ by excitation with the laser beam $L_2$. Therefore, the analyzing device 80 removes information about autofluorescence emitted by the cell $X_2$ from measured fluorescence data to calculate information about fluorescence emitted by the fluorescent protein $X_1$ by excitation with the laser beam $L_1$. More specifically, the analyzing device 80 stores the following previously-determined four sets of fluorescence data. The analyzing device 80 removes autofluorescence emitted by the cell $X_2$ from the measured fluorescence data, using the previously-determined fluorescence data and fluorescence data containing the Re components and the Im components supplied from the signal control unit 44, to calculate fluorescence data of fluorescence emitted by the fluorescent protein $X_1$.

The fluorescent protein $X_1$ and the cell $X_2$ are allowed to flow through the tube 30 of the flow cytometer 10 as separate samples, and the signal processing device 20 receives fluorescence (first fluorescence) emitted by the fluorescent protein $X_1$ within the wavelength band (first wavelength band) $R_1$ and the wavelength band (second wavelength band) $R_2$ and fluorescence (second fluorescence) emitted by the cell $X_2$ within the wavelength band (first wavelength band) $R_1$ and the wavelength band (second wavelength band) $R_2$. At this time, the signal processing device 20 performs mixing of fluorescent signals corresponding to the wavelength band $R_1$ with the modulation signal having a frequency of $f_1$, mixing of fluorescent signals corresponding to the wavelength band $R_2$ with the modulation signal having a frequency of $f_2$, and filtering of signals obtained by mixing, that is, the signal processing device 20 performs frequency-down conversion. As a result, the analyzing device 80 obtains four sets of fluorescence data containing information about phase and intensity. More specifically, fluorescence within the wavelength band $R_1$ emitted by the fluorescent protein $X_1$ is received, and then a fluorescent signal of the fluorescence is mixed with the modulation signal having a frequency of $f_1$ to produce fluorescence data. The thus produced fluorescence data (third fluorescence data) is defined as $A_3$. Fluorescence within

the wavelength band $R_2$ emitted by the fluorescent protein $X_1$ is received, and then a fluorescent signal of the fluorescence is mixed with the modulation signal having a frequency of $f_2$ to produce fluorescence data. The thus produced fluorescence data (fifth fluorescence data) is defined as $A_5$. Further, fluorescence within the wavelength band $R_1$ emitted by the cell $X_2$ is received, and then a fluorescent signal of the fluorescence is mixed with the modulation signal having a frequency of $f_1$ to produce fluorescence data. The thus produced fluorescence data (fourth fluorescence data) is defined as $A_4$. Fluorescence within the wavelength band $R_2$ emitted by the cell $X_2$ is received, and then a fluorescent signal of the fluorescence is mixed with the modulation signal having a frequency of $f_2$ to produce fluorescence data. The thus produced fluorescence data (sixth fluorescence data) is defined as $A_6$. These sets of fluorescence data $A_3$ to $A_6$ are each represented by a complex number having the values of the Re component and the Im component. These complex numbers are previously stored in the analyzing device 80.

It is to be noted that in this embodiment, the values of the fluorescence data $A_3$ to $A_6$ are determined by allowing the fluorescent protein $X_1$ and the cell $X_2$ to flow through the tube 30 as separate samples. However, the values of the fluorescence data $A_3$ to $A_6$ to be previously stored in the analyzing device 80 may be determined by accurately measuring fluorescence data using a filter suitable for the fluorescent protein $X_1$ and a filter suitable for the cell $X_2$ and then correcting measurement results based on the characteristics of filters used in the flow cytometer 10. Alternatively, this measurement may be performed using another device.

In the state, fluorescence data corresponding to a frequency of $f_1$ supplied from the signal control unit 44, that is, fluorescence data represented by a complex number having the Re component and the Im component obtained by mixing with the modulation signal having a frequency of $f_1$ is defined as $P_1$. On the other hand, fluorescence data corresponding to a frequency of $f_2$ supplied from the signal control unit 44, that is, fluorescence data represented by a complex number having the Re component and the Im component obtained by mixing with the modulation signal having a frequency of $f_2$ is defined as $P_2$.

At this time, fluorescence data P of fluorescence from which autofluorescence emitted by the cell $X_2$ has been removed, that is, fluorescence emitted by the fluorescent protein $X_1$ is calculated by the following formula (1).

$$P = (A_3/A_4)/\{(A_3/A_4) - (A_5/A_6)\} \cdot P_1 - (A_3/A_6)/\{(A_3/A_4) - (A_5/A_6)\} \cdot P_2 \qquad (1)$$

In the formula (1), $(A_3/A_4)/\{(A_3/A_4) - (A_s/A_6)\}$ is a first constant for processing the first fluorescence data $P_1$ and $(A_3/A_6)/\{(A_3/A_4) - (A_s/A_6)\}$ is a second constant for processing the second fluorescence data $P_2$. According to the formula (1), fluorescence data P can be obtained by subtracting a result obtained by multiplying the fluorescence data $P_2$ by the second constant from a result obtained by multiplying the fluorescence data $P_1$ by the first constant. It is to be noted that the first constant and the second constant are determined from the fluorescence data $A_3$ to $A_6$.

The reason why the fluorescence data of autofluorescence can be removed using the above formula (1) is based on the following concept.

The sample 12 composed of the cell $X_2$ and the fluorescent protein $X_1$ attached to the cell $X_2$ generally emits not only fluorescence emitted by the fluorescent protein $X_1$ but also autofluorescence. Therefore, the fluorescence data $P_1$ produced by using the fluorescent signal corresponding to the wavelength band $R_1$ and the modulation signal having a frequency of $f_1$ is represented as an addition of fluorescence data $P_{1X1}'$ of fluorescence emitted by the fluorescent protein $X_1$ and fluorescence data $P_{1X2}'$ of autofluorescence emitted by the cell $X_2$. Similarly, the fluorescence data $P_2$ produced by using the fluorescent signal corresponding to the wavelength range $R_2$ and the modulation signal having a frequency of $f_2$ is represented as an addition of fluorescence data $P_{2x1}'$ of fluorescence emitted by the fluorescent protein $X_1$ and fluorescence data $P_{2X2}'$ of autofluorescence emitted by the cell $X_2$.

Further, every time the sample 12 passes through the measurement point on which the laser beams $L_1$ and $L_2$ are focused, fluorescence intensity varies depending on the position of the sample 12 in the width direction of the measurement point. For example, there is a case where a certain one of the samples 12 passes through the peripheral portion of the spot of irradiation light (i.e., through a region where the intensity of laser light is lower), but the next sample 12 passes through the central portion of the spot of irradiation light (i.e., through a region where the intensity of laser light is the highest).

When a variation coefficient representing such a variation in fluorescence intensity is defined as C (0 or more but 1 or less), fluorescence data, which is obtained when the sample 12 passes through the central portion of the spot of irradiation light and which corresponds to the fluorescence data $P_{1x1}'$, is defied as fluorescence data $P_{1X1}$, and fluorescence data, which is obtained when the sample 12 passes through the central portion of the spot of irradiation light and which corresponds to the fluorescence data $P_{1x2}'$, is defined as fluorescence data $P_{1X2}$, the fluorescence data $P_1$ and the fluorescence data $P_2$ are represented by the following formulas (2) and (3), respectively. It is to be noted that fluorescence emitted by the fluorescent protein $X_1$ is represented as $n \cdot P_{1x1}$ and $n \cdot P_{2X1}$ assuming that fluorescence is emitted by two or more (n) fluorescent proteins X, attached to one cell $X_2$.

$$P_1 = C \cdot (n \cdot P_{1X1} + P_{1X2}) \qquad (2)$$

$$P_2 = C \cdot (n \cdot P_{2X1} + P_{2X2}) \qquad (3)$$

In the formula (2), $P_{1x1}$ and $P_{1X2}$ correspond to the above-mentioned fluorescence data $A_3$ and fluorescence data $A_4$. In the formula (3), $P_{2X1}$ and $P_{2X2}$ correspond to the above-mentioned fluorescence data $A_5$ and fluorescence data $A_6$. The values of the fluorescence data $A_3$ to $A_6$ are previously stored in the analyzing device 80. Therefore, C and n can be determined using the formulas (2) and (3). On the other hand, fluorescence data of fluorescence emitted by the fluorescent protein $X_1$ by excitation with the laser beam $L_1$, which should be calculated from the fluorescence data $P_1$, is $C \cdot n \cdot P_{1x1}$. When $C \cdot n \cdot P_{1X1}$ is represented using the fluorescence data $A_3$ to $A_6$, $P_1$, and $P_2$, the above formula (1) can be obtained.

According to the formula (1), it is possible to calculate fluorescence data of fluorescence emitted only by the fluorescent protein $X_1$ by removing autofluorescence in consideration of the fact that fluorescence intensity varies depending on the position of the sample 12 in the measurement point at the time when the sample 12 passes through the measurement point and there is a case fluorescence is emitted by two or more fluorochromes $X_1$ attached to one cell $X_2$.

The fluorescence intensity calculating unit 90 calculates the fluorescence intensity of the fluorescent protein $X_1$ by determining the absolute value of a complex number as for fluorescence data P of the fluorescent protein $X_1$ calculated by the autofluorescence removing unit 86.

The phase delay calculating unit 92 calculates a phase delay angle θ by determining the argument ($\tan^{-1}$(1m component of fluorescence data/Re component of fluorescence data)) of a complex number as for fluorescence data P of the fluorescent protein $X_1$ calculated by the autofluorescence removing unit 86.

The fluorescence relaxation time calculating unit 94 calculates the fluorescence relaxation time τ of the fluorescent protein $X_1$ by the following formula using the phase delay angle θ calculated by the phase delay calculating unit 92: $\tau = 1/(2\pi f_1) \cdot \tan(\theta)$, where $f_1$ is a frequency used to modulate the intensity of the laser beam $L_1$. The reason why the fluorescence relaxation time τ can be calculated by the formula $\tau = 1/(2\pi f_1) \cdot \tan(\theta)$ is that a fluorescence proceeds and changes itself according to a first-order relaxation process.

The calculated fluorescence intensity, phase delay angle θ, and fluorescence relaxation time τ of the fluorescent protein $X_1$ are outputted as result information to a printer or display (not illustrated). The result information is a result measured every time the sample 12 passes through the measurement point in the tube 30 and is subjected to statistical processing. The flow cytometer 10 has such a structure as described above.

Hereinbelow, a method for detecting fluorescence using the flow cytometer 10 will be described.

First, the light source unit 22 of the flow cytometer 10 emits, as irradiation light, the laser beams $L_1$ and $L_2$ different in wavelength while modulating the intensity of the laser beam $L_1$ at a frequency of $f_1$ and modulating the intensity of the laser beam $L_2$ at a frequency of $f_2$ different from $f_1$. It is to be noted that one of the laser beams $L_1$ and $L_2$ is prepared so that optical absorption of the fluorescent protein $X_1$ is higher than that of the cell $X_2$, and the other laser beam is prepared so that optical absorption of the cell $X_2$ is higher than that of the fluorescent protein $X_1$.

Then, the light-receiving unit 26 receives fluorescence within the wavelength band $R_1$ and fluorescence within the wavelength band $R_2$ and outputs fluorescent signals.

At this time, the ratio of fluorescence intensity between fluorescence within the wavelength band $R_1$ emitted by the fluorescent protein $X_1$ and fluorescence within the wavelength band $R_1$ emitted by the cell $X_2$ is preferably different from the ratio of fluorescence intensity between fluorescence within the wavelength band $R_2$ emitted by the fluorescent protein $X_1$ and fluorescence within the wavelength band $R_2$ emitted by the cell $X_2$. For example, the wavelength bands $R_1$ and $R_2$ are preferably set so that the fluorescence intensity of fluorescence within the wavelength band $R_1$ emitted by one of the fluorescent protein $X_1$ and the cell $X_2$ is higher than that of fluorescence within the wavelength band $R_1$ emitted by the other and the fluorescence intensity of fluorescence within the wavelength band $R_2$ emitted by the one of the fluorescent protein $X_1$ and the cell $X_2$ is lower than that of fluorescence within the wavelength band $R_2$ emitted by the other.

The control/processing unit 28 mixes one of the outputted fluorescent signals with the modulation signal for modulating the intensity of the laser beam $L_1$ to produce fluorescence data $P_1$ containing the phase delay angle of the fluorescent signal with respect to the modulation signal and the intensity amplitude of the fluorescent signal, and mixes the other outputted fluorescent signal with the modulation signal for modulating the intensity of the laser beam $L_2$ to produce fluorescence data $P_2$ containing the phase delay angle of the fluorescent signal with respect to the modulation signal and the intensity amplitude of the fluorescent signal.

The analyzing device 80 calculates fluorescence data P from the produced fluorescence data $P_1$ and $P_2$ by the above formula (1). The fluorescence data P is fluorescence data of fluorescence emitted by the fluorescent protein $X_1$, that is, fluorescence data from which autofluorescence emitted by the cell $X_2$ has been removed. Further, the analyzing device 80 calculates, by using the fluorescence data P, the fluorescence intensity, phase delay angle θ, and fluorescence relaxation time τ of fluorescence emitted by the fluorescent protein $X_1$.

It is to be noted that the values of the fluorescence data $A_3$ to $A_6$ used in the above formula (1 ) are previously determined by measuring the fluorescent protein $X_1$ and the cell $X_2$ separately or together by the flow cytometer 10 and stored in the analyzing device 80.

As has been described above, the analyzing device 80 can remove fluorescence data of autofluorescence emitted by the cell $X_2$ from measured fluorescence data prior to calculation of a fluorescence relaxation time τ, which makes it possible to calculate a fluorescence relaxation time τ with high accuracy.

According to this embodiment, two laser beams different in wavelength are employed, and absorption of one of the laser beams by a fluorochrome is lower than absorption of the other laser beam by the fluorochrome, which makes it possible to remove autofluorescence emitted by an analyte. At this time, a first constant and a second constant used to calculate fluorescence data are preferably determined by using four sets of fluorescence data obtained by measuring the fluorochrome and the analyte separately. In this case, the first constant, the second constant, and fluorescence data $P_1$ and $P_2$ obtained under the same laser excitation conditions and the same light-receiving conditions are used, which makes it possible to remove a factor that varies fluorescence data, that is, variation in fluorescence intensity depending on the position of a measurement object in the measurement point at the time when the measurement object passes through the measurement point.

Although the fluorescence detecting device and the fluorescence detecting method according to the present invention have been described above in detail, the present invention is not limited to the above embodiment, and it should be understood that various changes and modifications may be made without departing from the scope of the present invention.

**Claims**

1.  A device for detecting fluorescence by processing a fluorescent signal of fluorescence emitted by a measurement object, which is an analyte having at least one fluorochrome attached thereto, by irradiation with laser light, the device comprising:

    a light source unit configured to modulate an intensity of a first laser beam by a modulation signal having a frequency of $f_1$ and to modulate an intensity of a second laser beam by a modulation signal having a frequency of $f_2$ different from $f_1$ to emit the modulated first laser beam and the modulated second laser beam;
    a light-receiving unit configured to include a first light-receiving element for receiving, within a first wavelength band, fluorescence emitted by the measurement object irradiated with the first laser beam and the second laser beam to output a first fluorescent signal and a second light-receiving element that receives, within a second wavelength band different from the first wavelength band, fluorescence emitted by the measurement object irradiated with the first laser beam and the second laser beam to output a second fluorescent signal;
    a first processing unit configured to produce, by mixing the first fluorescent signal with the modulation signal having a frequency of $f_1$, first fluorescence data $P_1$ containing information about phase and intensity and to produce, by mixing the second fluorescent signal with the modulation signal having a frequency of $f_2$, second fluorescence data $P_2$ containing information about phase and intensity; and
    a second processing unit configured to calculate a fluorescence relaxation time of the fluorochrome using fluorescence data obtained by subtracting a result obtained by multiplying the second fluorescence data $P_2$ by a second constant from a result obtained by multiplying the first fluorescence data $P_1$ by a first constant.

2.  The device according to claim 1, wherein the first laser beam has a wavelength within a wavelength band in which optical absorption of the fluorochrome is higher than that of the analyte and the second laser beam has a wavelength within a wavelength band in which optical absorption of the fluorochrome is lower than that of the analyte.

3.  The device according to claim 1 or 2, wherein a ratio of fluorescence intensity between fluorescence within the first wavelength band emitted by the fluorochrome and fluorescence within the first wavelength band emitted by the analyte is different from a ratio of fluorescence intensity between fluorescence within the second wavelength band emitted by the fluorochrome and fluorescence within the second wavelength band emitted by the analyte.

4. The device according to any one of claims 1 to 3, wherein the second processing unit is previously configured to store two sets of fluorescence data $A_3$ and $A_4$ containing information about phase and intensity produced by mixing the modulation signal having a frequency of $f_1$ with two fluorescent signals generated by separately receiving first fluorescence within the first wavelength band emitted by the fluorochrome and second fluorescence within the first wavelength band emitted by the analyte, and is further previously configured to store two sets of fluorescence data $A_5$ and $A_6$ containing information about phase and intensity produced by mixing the modulation signal having a frequency of $f_2$ with two fluorescent signals generated by separately receiving first fluorescence within the second wavelength band emitted by the fluorochrome and second fluorescence within the second wavelength band emitted by the analyte, and is configured to determine the first constant and the second constant using the fluorescence data $A_3$, $A_4$, $A_5$, and $A_6$ stored therein to calculate the fluorescence relaxation time.

5. The device according to claim 2, wherein the second processing unit is configured to calculate a fluorescence relaxation time using fluorescence data P calculated by a following formula using the fluorescence data $A_3$, $A_4$, $A_5$, and $A_6$:

$$P = (A_3/A_4)/\{(A_3/A_4) - (A_5/A_6)\} \cdot P_1 - (A_3/A_6)/\{(A_3/A_4) - (A_5/A_6)\} \cdot P_2$$

6. The device according to any one of claims 1 to 5, wherein fluorescence emitted by the measurement object includes fluorescence emitted by the fluorochrome and autofluorescence emitted by the analyte itself.

7. The device according to claim 6, wherein the analyte is a biological material.

8. A method for detecting fluorescence by processing a fluorescent signal of fluorescence emitted by a measurement object, which is an analyte having a fluorochrome attached thereto, by irradiation with laser light, the method comprising the steps of:

modulating an intensity of a first laser beam by a modulation signal having a frequency of $f_1$ and modulating an intensity of a second laser beam by a modulation signal having a frequency of $f_2$ different from $f_1$ to emit the modulated first laser beam and the modulated second laser beam;
receiving, within a first wavelength band, fluorescence emitted by the measurement object irradiated with the first laser beam and the second laser beam to output a first fluorescent signal and receiving, within a second wavelength band different from the first wavelength band, fluorescence emitted by the measurement object irradiated with the first laser beam and the second laser beam to output a second fluorescent signal;
producing, by mixing the first fluorescent signal with the modulation signal having a frequency of $f_1$, first fluorescence data $P_1$ containing information about phase and intensity and producing, by mixing the second fluorescent signal with the modulation signal having a frequency of $f_2$, second fluorescence data $P_2$ containing information about phase and intensity; and
calculating a fluorescence relaxation time of the fluorochrome using fluorescence data obtained by subtracting a result obtained by multiplying the second fluorescence data $P_2$ by a second constant from a result obtained by multiplying the first fluorescence data $P_1$ by a first constant.

9. The method according to claim 8, wherein the first laser beam has a wavelength within a wavelength band in which optical absorption of the fluorochrome is higher than that of the analyte and the second laser beam has a wavelength within a wavelength band in which optical absorption of the fluorochrome is lower than that of the analyte.

10. The method according to claim 8 or 9, wherein a ratio of fluorescence intensity between fluorescence within the first wavelength band emitted by the fluorochrome and fluorescence within the first wavelength band emitted by the analyte is different from a ratio of fluorescence intensity between fluorescence within the second wavelength band emitted by the fluorochrome and fluorescence within the second wavelength band emitted by the analyte.

11. The method according to any one of claims 8 to 10, wherein the first constant and the second constant are determined using previously-obtained four sets of fluorescence data, the method further comprising the steps of:

determining, by mixing the modulation signal having a frequency of $f_1$ with two fluorescent signals generated by separately receiving first fluorescence within the first wavelength band emitted by the fluorochrome and second fluorescence within the first wavelength band emitted by the analyte, two sets of fluorescence data $A_3$

and $A_4$ containing information about phase and intensity and storing the two sets of fluorescence data $A_3$ and $A_4$; and

determining, by mixing the modulation signal having a frequency of $f_2$ with two fluorescent signals generated by separately receiving first fluorescence within the second wavelength band emitted by the fluorochrome and second fluorescence within the second wavelength band emitted by the analyte, two sets of fluorescence data $A_5$ and $A_6$ containing information about phase and intensity and storing the two sets of fluorescence data $A_5$ and $A_6$.

**12.** The method according to claim 11, wherein the second processing unit calculates a fluorescence relaxation time using fluorescence data P calculated by a following formula using the fluorescence data $A_3$, $A_4$, $A_5$, and $A_6$:

$$P = (A_3/A_4)/\{(A_3/A_4) - (A_5/A_6)\} \cdot P_1 \quad - (A_3/A_6)/\{(A_3/A_4) - (A_5/A_6)\} \cdot P_2$$

FIG.1

FIG.2

**FIG.3**

FIG.4

FIG.5

<table>
<tr><td colspan="2" align="center">**INTERNATIONAL SEARCH REPORT**</td><td colspan="2">International application No.<br>PCT/JP2010/000002</td></tr>
</table>

A.  CLASSIFICATION OF SUBJECT MATTER
*G01N21/64*(2006.01)i

According to International Patent Classification (IPC) or to both national classification and IPC

B.  FIELDS SEARCHED

Minimum documentation searched (classification system followed by classification symbols)
G01N21/62-G01N21/83, G01N33/48-G01N33/98, G01N15/00-G01N15/14

Documentation searched other than minimum documentation to the extent that such documents are included in the fields searched
Jitsuyo Shinan Koho          1922-1996   Jitsuyo Shinan Toroku Koho   1996-2010
Kokai Jitsuyo Shinan Koho    1971-2010   Toroku Jitsuyo Shinan Koho   1994-2010

Electronic data base consulted during the international search (name of data base and, where practicable, search terms used)
JSTPlus(JDreamII), JMEDPlus(JDreamII), JST7580(JDreamII)

C.  DOCUMENTS CONSIDERED TO BE RELEVANT

| Category* | Citation of document, with indication, where appropriate, of the relevant passages | Relevant to claim No. |
|---|---|---|
| A | JP 2007-127415 A  (Mitsui Engineering & Shipbuilding Co., Ltd.), 24 May 2007 (24.05.2007), claim 1; paragraphs [0022] to [0023], [0033] to [0065] & US 2009/0012721 A1    & EP 1855102 A1 & WO 2006/088047 A1 | 1-12 |
| A | JP 2007-240424 A  (Mitsui Engineering & Shipbuilding Co., Ltd.), 20 September 2007 (20.09.2007), claims 1, 8 (Family: none) | 1-12 |

☒  Further documents are listed in the continuation of Box C.          ☐   See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>03 February, 2010 (03.02.10) | Date of mailing of the international search report<br>16 February, 2010 (16.02.10) |
|---|---|
| Name and mailing address of the ISA/<br>Japanese Patent Office | Authorized officer |
| Facsimile No. | Telephone No. |

Form PCT/ISA/210 (second sheet) (April 2007)

**EP 2 383 564 A1**

| INTERNATIONAL SEARCH REPORT | International application No. |
|---|---|
| | PCT/JP2010/000002 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2004-251814 A (Kabushiki Kaisha Oputokuesuto), 09 September 2004 (09.09.2004), paragraph [0034] (Family: none) | 1-12 |
| A | JP 2002-533658 A (Photosense, L.L.C.), 08 October 2002 (08.10.2002), claim 1 & US 6157037 A         & EP 1144909 A & WO 2000/037850 A1     & AU 3996000 A & BR 9916948 A         & CA 2353421 A | 1-12 |
| A | JP 2004-163312 A (Arkray, Inc.), 10 June 2004 (10.06.2004), claims 1, 3 & US 2006/0108540 A1     & EP 1562037 A1 & WO 2004/044564 A1     & CN 1711469 A | 1-12 |

Form PCT/ISA/210 (continuation of second sheet) (April 2007)

19

**EP 2 383 564 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006226698 A